# EUROPÄISCHE PATENTANMELDUNG

(11) **EP 0 770 620 A1**
(43) Veröffentlichungstag der Anmeldung: **02.05.1997**
(21) Anmeldenummer: 96116672.5
(22) Anmeldetag: 17.10.1996
(51) Int. Cl.: C07H 13/06, C07H 15/04, C07C 219/06, C07C 217/08, C11D 1/62, A61K 7/06, A61K 7/08, A61K 7/48, A61K 7/50

(54) **Glucoquats und ihre Verwendung als oberflächenaktive Mittel insbesondere zur Haar- und Körperpflege**

(30) Priorität: 26.10.1995 DE 19539845
(71) Anmelder: Henkel Kommanditgesellschaft auf Aktien, 40589 Düsseldorf-Holthausen (DE)
(72) Erfinder: Pi Subirana, Rafael, Dr., 08400 Granollers (ES); Bigorra Llosas, Joaquim, Dr., 08203 Sadabell (ES)

(57) **Zusammenfassung**

Es werden neue Esterquats auf Zuckerbasis vorgeschlagen, die man erhält, indem man Trialkanolamine mit einer Mischung aus Zuckersäuren und Fettsäuren umsetzt, die resultierenden Ester gegebenenfalls alkoxyliert und anschließend in an sich bekannter Weise quaterniert. Es werden kationische Tenside erhalten, die eine besonders hohe ökotoxikologische Verträglichkeit besitzen, synthetischen wie natürlichen Fasern einen angenehmen Griff verleihen und dabei gleichzeitig die elektrostatische Aufladung zwischen den Filamen-ten herabsetzen.

## Beschreibung

### Gebiet der Erfindung

Die Erfindung betrifft neue zuckerbasierte Esterquats, die man erhält, indem man Trialkanolamine mit Zucker- und Fettsäuren umsetzt und die resultierenden Ester - gegebenenfalls nach Alkoxylierung - in an sich bekannter Weise quaterniert.

### Stand der Technik

Unter der Bezeichnung 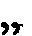 Esterquats 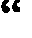 werden im allgemeinen quaternierte Fettsäuretriethanolaminestersalze verstanden, die sich in breitem Umfang sowohl für die Faser- als auch für die Haaravivage eignen. In den vergangenen Jahren haben diese Stoffe infolge ihrer besseren ökotoxikologischen Verträglichkeit konventionelle quartäre Ammoniumverbindungen wie z.B. das bekannte Distearyldimethylammoniumchlorid zu einem guten Teil vom Markt verdrängt. Übersichten zu diesem Thema sind beispielsweise von O.Ponsati in **C.R. CED-Kongress, Barcelona, 1992, S.167 ,** R.Puchta et al. in **Tens.Surf.Det., 30, 186 (1993),** M.Brock in **Tens. Surf. Det. 30, 394 (1993)** und R.Lagerman et al. in **J.Am.Oil.Chem.Soc., 71, 97 (1994)** erschienen.

Kationische Avivagemittel auf Basis von Aminozuckern (Glucamin, Bis-Bissorbitylamin) sind ferner aus den Deutschen Offenlegungsschriften **DE-A1 42 38 207, DE 42 38 212, DE-A1 42 38 213, DE-A1 42 38 216** und **DE-A1 42 38 217** (Henkel) bekannt.

Obschon Esterquats des Stands der Technik über sehr gute anwendungstechnische Eigenschalten verfügen sowie eine zufriedenstellende biologische Abbaubarkeit und eine gute hautkosmetische Verträglichkeit besitzen, gehen die Anforderungen des Verbrauchers weiterhin in die Richtung verbesserter Produkteigenschaften.

Demzufolge hat die Aufgabe der Erfindung darin bestanden, neue Esterquat-Typen zur Verfügung zu stellen, die sich durch eine weiter verbesserte ökotoxikologische Verträglichkeit auszeichnen und gleichzeitig ein gutes Avivage - und Antistatikverhalten aufweisen.

### Beschreibung der Erfindung

Gegenstand der Erfindung sind Glucoquats, die man erhält, indem man Trialkanolamine mit einer Mischung aus Zuckersäuren und Fettsäuren umsetzt, die resultierenden Ester gegebennenfalls alkoxyliert und anschließend in an sich bekannter Weise quaterniert.

Durch Veresterung mit einer Mischung von Fettsäuren und Zuckersäuren werden neue zuckerbasierte kationische bzw. amphotere Esterquattenside erhalten, die sich überraschenderweise gegenüber Produkten des Stands der Technik nicht nur durch eine besonders gute ökotoxikologische Verträglichkeit, sondern auch ausgezeichnete Haar-und Faseravivage sowie eine Verminderung der elektrostatischen Aufladung zwischen den Faserfilamenten auszeichnen.

Ein weiterer Gegenstand der Erfindung betrifft ein Verfahren zur Herstellung von Glucoquats, bei dem man Trialkanolamine mit einer Mischung aus Zuckersäuren und Fettsäuren umsetzt und die resultierenden Ester gegebenenfalls alkoxyliert und anschließend in an sich bekannter Weise quaterniert.

### Trialkanolamine

Beispiele für Trialkanolamine, die im Sinne der Erfindung als zentrale Stickstoffverbindungen in Betracht kommen, sind in erster Linie Triethanolamin sowie Anlagerungsprodukte von 1 bis 10 und vorzugsweise 2 bis 5 Mol Ethylenoxid an diese Verbindungen.

### Zuckersäuren

Unter dem Begriff Zuckersäuren werden zur Lactonbildung neigende Polyhydroxy-carbonsäuren verstanden, die durch Oxidation der Aldehyd- bzw. primären Hydroxylgruppen von Monosacchariden zu Carboxylgruppen entstehen. Im Sinne der Erfindung kommen sowohl **Aldonsäuren** wie beispielsweise Gluconsäure, Glucarsäure oder Ascorbinsäure in Frage als auch **Uronsäuren** wie die Glucuronsäure oder die Galacturonsäure oder **Ketoaldonsäuren** wie beispielsweise die 2-Oxogluconsäure. Vorzugsweise werden Glucuronsäure, Ascorbinsäure oder Glucuronsäure eingesetzt.

### Fettsäuren

Unter Fettsäuren sind aliphatische Carbonsäuren der Formel (**I**) zu verstehen, 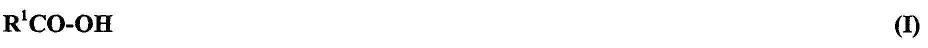 in der R¹CO für einen aliphatischen, linearen oder verzweigten Acylrest mit 6 bis 22 Kohlenstoffatomen und 0 und/oder 1, 2 oder 3 Doppelbindungen steht. Typische Beispiele sind Capronsäure, Caprylsäure, 2-Ethylhexansäure, Caprinsäure, Laurinsäure, Isotridecan-säure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gädoleinsäure, Behensäure und Erucasäure sowie deren technische Mischungen, die z.B. bei der Druckspaltung von natürlichen Fetten und Ölen, bei der Reduktion von Aldehyden aus der Roelen'schen Oxosynthese oder der Dimerisierung von ungesättigten Fettsäuren anfallen. Bevorzugt sind technische Fettsäuren mit 12 bis 18 Kohlenstoffatomen wie beispielsweise Kokos-, Palm-, Palmkern- oder Talgfettsäure, vorzugsweise in gehärteter bzw. teilgehärteter Form.

### Veresterung

Die Zuckersäuren und die Fettsäuren können im molaren Verhältnis von 1 : 10 bis 10 : 1 eingesetzt werden. Es hat sich jedoch als vorteilhaft erwiesen, ein molares Verhältnis von 1 : 4 bis 1 : 6 einzustellen. Die Trialkanolamine einerseits und die Säuren - also Zucker-und Fettsäuren zusammengenommen - können im molaren Verhältnis 1 : 1,3 bis 1 : 2,4 eingesetzt werden. Als optimal hat sich ein molares Verhältnis Trialkanolamin : Säuren von 1 : 1,4 bis 1 : 1,8 erwiesen.

Die Veresterung kann in an sich bekannter Weise durchgeführt werden, wie sie beispielsweise in der Internationalen Patentanmeldung **WO 91/01295** (Henkel) beschrieben wird. Vorteilhafterweise erfolgt die Veresterung bei Temperaturen von 120 bis 220 und insbesondere 130 bis 170°C und Drücken von 0,01 bis 1 bar. Als geeignete Katalysatoren haben sich hypophosphorige Säuren bzw. deren Alkalisalze, vorzugsweise Natriumhypophosphit bewährt, die in Mengen von 0,01 bis 0,1 und vorzugsweise 0,05 bis 0,07 Gew.-% - bezogen auf die Einsatzstoffe - eingesetzt werden können. Im Hinblick auf eine besonders hohe Farbqualität und - stabitität hat sich die Mitverwendung von Alkali- und/oder Erdalkaliborhydriden, wie beispielsweise Kalium-, Magnesium- und insbesondere Natriumborhydrid als vorteilhaft erwiesen. Die Co-Katalysatoren setzt man üblicherweise in Mengen von 50 bis 1000 und insbesondere 100 bis 500 ppm - wieder bezogen auf die Einsatzstoffe - ein. Entsprechende Verfahren sind auch Gegenstand der beiden Deutschen Patentschriften **DE-C1 43 08 792** und **DE-C1 44 09 322** (Henkel), auf deren Lehren hiermit ausdrücklich Bezug genommen wird.

Es ist möglich, Mischungen der Zucker- und Fettsäuren einzusetzen oder aber die Veresterung mit den beiden Komponenten nacheinander durchzuführen. In einer besonders vorteilhatten Ausführungsform der Erfindung führt man zunächste eine partielle Veresterung der Trialkanolamine mit den Zuckersäuren durch, ehe man anschließend die Fettsäuren zugibt.

### Alkoxylierung

Zur Herstellung von polyalkylenoxidhaltigen Glucoquats kann man nach zwei Alternativen verfahren. Zum einen kann man ethoxylierte Trialkanolamine einsetzen. Dies hat den Vorteil, daß die Alkylenoxidverteilung im später resultierenden Glucoquat bezüglich der drei OH-Gruppen des Amins annähernd gleich ist. Nachteilig ist jedoch, daß die Veresterung aus sterischen Gründen schwieriger wird. Die Methode der Wahl besteht daher darin, den Ester vor der Quaternierung zu alkoxylieren. Dies kann in an sich bekannter Weise geschehen, d.h. in Anwesenheit basischer Katalysatoren und bei erhöhten Temperaturen. Als Katalysatoren kommen beispielsweise Alkali- und Erdalkalihydroxide und -alkoholate, vorzugsweise Natriumhydroxid und insbesondere Natriummethanolat in Betracht; die Einsatzmenge liegt üblicherweise bei 0,5 bis 5 und vorzugsweise 1 bis 3 Gew.-% - bezogen auf die Einsatzstoffe. Bei Verwendung dieser Katalysatoren werden in erster Linie freie Hydroxylgruppen alkoxyliert. Setzt man als Katalysatoren jedoch calcinierte oder mit Fettsäuren hydrophobierte Hydrotalcite ein, kommt es auch zu einer Insertion der Alkylenoxide in die Esterbindungen. Diese Methode ist bevorzugt, wenn man eine Alkylenoxidverteilung wünscht, die der bei Einsatz von alkoxylierten Trialkanolaminen nahe kommt. Als Alkylenoxide können Ethylen-und Propylenoxid sowie deren Gemische (Random- oder Blockverteilung) eingesetzt werden. Die Reaktion wird üblicherweise bei Temperaturen im Bereich von 100 bis 180°C durchgeführt. Wegen des empfindlichen Zuckeranteils ist es von Vorteil, bei möglichst niedrigen Temperaturen beispielsweise im Bereich von 110 bis 140°C zu arbeiten und dafür eine etwas längere Reaktionszeit in Kauf zu nehmen. Durch den Einbau von im Durchschnitt 1 bis 10 Mol Alkylenoxid pro Mol Ester wird die Hydrophilie der Glucoquats gesteigert, die Löslichkeit verbessert und die Reaktivität gegenüber anionischen Tensiden herabgesetzt.

### Quaternierung und Alkylierungsmittel

Die Quaternierung der Zucker/Fettsäure-trialkanolaminester kann in an sich bekannter Weise durchgeführt werden. Obschon die Umsetzung mit den Alkylierungsmitteln auch in Abwesenheit von Lösungsmitteln durchgeführt werden kann, empfiehlt sich die Mitverwendung zumindest von geringen Mengen Wasser oder niederen Alkoholen, vorzugsweise Isopropylalkohol, zur Herstellung von Konzentraten, die einen Feststoffanteil von mindestens 85 und insbesondere mindestens 90 Gew.-% aufweisen.

Als Alkylierungsmittel kommen Alkylhalogenide wie beispielsweise Methylchlorid, Dialkylsulfate wie beispielsweise Dimethylsulfat oder Diethylsulfat, oder Dialkylcarbonate wie beispielsweise Dimethylcarbonat oder Diethylcarbonat in Frage.

Üblicherweise werden die Ester und die Alkylierungsmittel im molaren Verhältnis 1 : 0,95 bis 1 : 1,05, also annähernd stöchiometrisch eingesetzt. Die Reaktionstemperatur liegt gewöhn-lich bei 40 bis 80 und insbesondere bei 50 bis 60°C. Im Anschluß an die Reaktion empfiehlt es sich, nichtumgesetztes Alkylierungsmittel durch Zugabe beispielsweise von Ammoniak, einem (Alkanol)amin, einer Aminosäure oder einem Oligopeptid zu zerstören, wie dies beispielsweise in der Deutschen Patentanmeldung **DE-A1 40 26 184** (Henkel) beschrieben wird.

### Dispergatoren und Emulgatoren

Üblicherweise wird die Quaternierung entweder wasserfrei oder in Gegenwart von geringen Mengen eines Lösungsmittels (z.B. Isopropylalkohol) durchgeführt. Je nach Einsatzzweck der Glucoquats kann es jedoch vorteilhaft sein, einen zukünftig mitzuverwendenen Dispergator oder Emulgator in das Reaktionsprodukt einzubauen, d.h. auf das eigentliche Lösungsmittel zu verzichten, das ja eigentlich nur dazu dient, eine flüssige Phase herzustellen, und die Quaternierung in Gegenwart des Dispergators/Emulgators als Solvens durchzuführen. Ein entsprechendes Verfahren ist beispielsweise in den Deutschen Patentschritten **DE-C1 43 08 794, DE-C1 43 35 782** und **DE-C1 43 39 643** (Henkel) beschrieben.

Als Dispergatoren und/oder Emulgatoren kommen beispielsweise **Fettalkohole** in Betracht. Typische Vertreter sind Capronalkohol, Caprylalkohol, 2-Ethylhexylalkohol, Caprinalko-hol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Bevorzugt sind technische Fettalkohole mit 12 bis 18 Kohlenstoffatomen wie beispielsweise Kokos-, Palm-, Palmkern- oder Talgfettalkohol.

Weiterhin sind als Dispergatoren und/oder Emulgatoren **Polyole** geeignet. Typische Vertreter sind
- Glycerin;
- Alkylenglycole wie beispielsweise Ethylenglycol, Diethylenglycol, Propylenglycol;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche, mit 1 bis 8 Kohlenstoffen im Alkylrest wie beispielsweise Methyl- und Butylglucosid;
- Zuckeralkohole mit 5 bis 12 Kohlenstoffatomen wie beispielsweise Sorbit oder Mannit,
- Zucker mit 5 bis 12 Kohlenstoffatomen wie beispielsweise Glucose oder Saccharose;
- Aminozucker wie beispielsweise Glucamin.

Als weitere Gruppe von geeigneten Dispergatoren und/oder Emulgatoren sind Partialglyceride wie z.B. Mono- und/oder Diglyceride sowie anionische und nichtionische Tenside zu nennen. Unter den nichtionischen Tensiden ist der Einsatz von Alkyloligoglucosiden, Fettsäure-N-alkylglucamiden und/oder Anlagerungsprodukten von durchschnittlich 1 bis 50 Mol Ethylenoxid an die oben genannten Fettalkohole besonders bevorzugt. Das Gewichtsverhältnis Ester zu Dispergator/Emulgator kann im Bereich 30 : 70 bis 70 : 30 liegen.

### Tenside

Die erfindungsgemäßen Glucoquats können zusammen mit weiteren anionischen, nichtionischen, kationischen und/oder amphoteren Tensiden eingesetzt werden. Wegen des Problems der Adduktbildung zwischen kationischen und anionischen Tensiden sind natürlich Mischungen der Glucoquats mit nichtionischen, amphoteren und zwitterionischen Tensiden bevorzugt. Glucoquats, insbesondere solche, die über Polyoxyalkylengruppen verfügen, besitzen jedoch gegenüber anionischen Tensiden eine vergleichsweise stark herabgesetzte Reaktivität, so daß das Problem der Salzbildung und/oder Inaktivierung in der Praxis kaum zum Tragen kommt.

Typische Beispiele für **anionische Tenside** sind Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Hydroxymischether-sulfate, Monoglycerid(ether)sulfate, Fettsäureamid(ether)sulfate, Mono- und Dialkylsul-fosuccinate, Mono- und Dialkylsulfosuccinamate, Sulfotriglyceride, Amidseifen, Ether-carbonsäuren und deren Salze, Fettsäureisethionate, Fettsäuresarcosinate, Fettsäuretauride, Acyllactylate, Acyltartrate, Acylglutamate, Acylaspartate, Alkyloligoglucosidsulfate, Protein-fettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen.

Typische Beispiele für **nichtionische Tenside** sind Fettalkoholpolyglycolether, Alkylphenolpolyglycolether, Fettsäurepolyglycolester, Fettsäureamidpolyglycolether, Fettaminpolygly-colether, alkoxylierte Triglyceride, Mischether bzw. Mischformale, Alk(en)yloligoglykoside, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen.

Typische Beispiele für **kationische Tenside** sind quartäre Ammoniumverbindungen und Esterquats, insbesondere quaternierte Fettsäuretrialkanolaminester-Salze.

Typische Beispiele für **amphotere bzw. zwitterionische Tenside** sind Alkylbetaine, Alkylamidobetaine, Aminopropionate, Aminoglycinate, Imidazoliniumbetaine und Sulfobetaine.

Bei den genannten Tensiden handelt es sich ausschließlich um bekannte Verbin-dungen. Hinsichtlich Struktur und Herstellung dieser Stoffe sei auf einschlägige Übersichtsarbeiten beispielsweise **J.Falbe (ed.), "Surfactants in Consumer Products", Springer Verlag, Ber-in, 1987, S. 54-124** oder **J.Falbe (ed.), "Katalysatoren, Tenside und Mineralöladditive", Thieme Verlag, Stuttgart, 1978, S. 123-217** verwiesen. Aus anwendungstechnischer Sicht sind Abmischungen von Glucoquats mit den genannten Tensiden im Gewichtsverhältnis 10 : 90 bis 90 : 10 bevorzugt. Besonders vorteilhafte Eigenschaften werden bei Kombinationen von Glucoquats mit Alkyloligoglucosiden, Fettsäure-N-alkylglucamiden und/oder Betainen erhalten.

### Gewerbliche Anwendbarkeit

Die erfindungsgemäßen Glucoquats weisen ein ausgezeichnetes Avivagevermögen auf, sind bei Vorliegen einer Betainstruktur schaumstark und reinigungsstark, vermindern als Kationtenside die elektrostatische Aufladung zwischen synthetischen und natürlichen Fasern, auch Keratinfasern, und zeichnen sich durch besonders vorteilhafte ökotoxikologische Verträglichkeit aus.

Ein weiterer Gegenstand der Erfindung betrifft daher ihre Verwendung zur Herstellung von oberflächenaktiven Mitteln wie z.B. Avivagemitteln sowie vorzugsweise Mitteln zur Haar-und Körperpflege, in denen sie in Mengen von 1 bis 50, vorzugsweise 3 bis 35 Gew.-% - bezogen auf die Mittel - enthalten sein können.

### Haar- und Körperpflegemittel

Die Haar- und Körperpflegemittel, wie beispielsweise Haarshampoos, Haarlotionen oder Dusch- und Schaumbäder, können als weitere Hilfs- und Zusatzstoffe Emulgatoren, Überfettungsmittel, Verdickungsmittel, Kationpolymere, Siliconverbindungen, biogene Wirkstoffe, Filmbildner, Konservierungsmittel, Farb- und Duftstoffe enthalten.

Als **Emulgatoren** kommen sowohl bekannte W/O- als auch O/W- Emulgatoren wie beispielsweise gehärtetes und ethoxyliertes Ricinusöl Polyglycerinfettsäureester oder Polyglycerinpolyricinoleate bzw. Polyglycerinpoly-12-hydroxystearate in Frage. Als **Überfettungsmittel** können Substanzen wie beispielsweise polyethoxylierte Lanolinderivate, Lecithinderivate, Polyolfettsäureester, Monoglyceride und Fettsäurealkanolamide verwendet werden, wobei die letzteren gleichzeitig als Schaumstabilisatoren dienen. Geeignete **Verdickungsmittel** sind beispielsweise Polysaccharide, insbesondere Xan-than-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, Polyvinylalkohol und Polyvinylpyrrolidon, Tenside wie beispielsweise Fettalkoholethoxylate mit eingeengter Homologenverteilung oder Alkyloligoglucoside sowie Elektrolyte wie Kochsalz und Ammoniumchlorid.

Geeignete **kationische Polymere** sind beispielsweise kationischen Cellulosederivate, kationischen Stärke, Copolymere von Diallylammoniumsalzen und Acrylamiden, quaternierte Vinylpyrrolidor/Vinylimidazol-Polymere wie z.B. Luviquat® (BASF AG, Ludwigshafen/FRG), Kondensationsprodukte von Polyglycolen und Aminen, quaternierte Kollagenpolypeptide wie beispielsweise Lauryldimonium hydroxypropyl hydrolyzed collagen (Lamequat®L, Grünau GmbH), Polyethylenimin, kationische Siliconpolymere wie z.B. Amidomethicone oder Dow Corning, Dow Corning Co./US, Copolymere der Adipinsäure und Dimethylaminohydroxypropyldiethylentrimamin (Cartaretine®, Sandoz/CH), Polyaminopolyamide wie z.B. beschrieben in der **FR-A 22 52 840** sowie deren vernetzte wasserlöslichen Polymere, kationische Chitinderivate wie beispielsweise quaterniertes Chitosan, gegebenenfalls mikrokristallin verteilt, kationischer Guar-Gum wie z.B. Jaguar® CBS, Jaguar® C-17, Jaguar® C-16 der Celanese/US, quaternierte Ammoniumsalz-Polymere wie z.B. Mirapol® A-15, Mirapol® AD-1, Mirapol® AZ-1 der Miranol/US.

Geeignete **Siliconverbindungen** sind beispielsweise Dimethylpolysiloxane, Methylphenylpolysiloxane, cyclische Silicone sowie amino-, fettsäure-, alkohol-, polyether-, epoxy-, fluor- und/oder alkylmodifizierte Siliconverbindungen. Unter **biogenen Wirkstoffen** sind beispielsweise Pflanzenextrakte und Vitaminkomplexe zu verstehen. Gebräuchliche **Filmbildner** sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen. Als **Konservierungsmittel** eignen sich beispielsweise Phenoxyethanol, Formaldehydlösung, Parabene, Pentandiol oder Sorbinsäure. Als **Perlglanzmittel** kommen beispielsweise Glycoldistearinsäureester wie Ethylenglycoldistearat, aber auch Fettsäuremonoglycolester in Betracht. Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen.

### Beispiele

### I. Herstellbeispiele

### Beispiel 1

**Glucoquats auf Basis Gluconsäure.** In einem 1-l-Dreihalskolben mit Rührer, Innenthermometer und Destillationsaufsatz wurden 280 g (1,88 mol) Triethanolamin, 221 g (0,56 mol) Gluconsäure (50 Gew.-%ig in Wasser) und 0,8 g - entsprechend 0,7 Gew.-% bezogen auf die Einsatzstoffe - Natriumhypophosphit vorgelegt und auf 175°C erhitzt.

200 g des resultierenden Esters wurden in eine Vakuumrührapparatur überführt und mit 400 g (1,48 mol) teilgehärteter Palmfettsäure und 0,6 g Natriumhypophosphit (50 Gew.-%ig in Wasser) versetzt. Die Mischung wurde unter Durchleiten von Stickstoff zunächst auf 70°C erhitzt, dann wurde der Druck stufenweise zunächst auf 25 und dann bis auf 2 mbar vermindert und die Temperatur langsam bis auf 160°C gesteigert.

476 g (0,82 mol) des resultierenden Esters wurden anschließend bei 40°C in 65 ml Isopropylalkohol gelöst, auf 60°C erhitzt und über einen Zeitraum von 2 h mit 110 g (0,87 mol) Dimethylsulfat versetzt. Nach Beendigung der Zugabe ließ man noch weitere 4 h bei 60°C rühren. Anschließend wurde die resultierende dunkelgefärbte Paste auf einen pH-Wert von 2,5 eingestellt. Das Produkt wies folgende Kennzahlen auf:

| | |
|---|---|
| Aktivsubstanz | 1,27 meq/g |
| Trockenrückstand | 92,9 Gew.-% |
| Stickstoff | 0,047 meq/g |
| Säurezahl | 10,8 |

### Beispiel 2

235 g (0,5 mol) des rohen Fettsäure/Gluconsäure-triethanolaminesters aus Beispiel 1 wurden wieder in einer Quaternierungsapparatur vorgelegt und dort bei 45°C mit 100 g Kokosfettalkohol zu einer homogenen Masse verrührt. Anschließend wurde wie in Beispiel 1 beschrieben, jedoch ohne Zusatz von Isopropylalkohol, quaterniert. Es resultierte eine beigegefärbte, wachsartige Masse, die sich leicht zu Schuppen verarbeiten ließ.

### Beispiel 3

Beispiel 2 wurde wiederholt, anstelle des Kokosfettalkohols jedoch 100 g einer 35 Gew.-%igen wäßrigen Paste eines Alkyloligoglucosids (Plantaren® APG 2000, Henkel KGaA, Düsseldorf/FRG) eingesetzt. Es resultierte eine hellbeigegefärbte Paste.

### Beispiel 4

Beispiel 2 wurde wiederholt, anstelle des Kokosfettalkohols jedoch 100 g eines Gemisches aus einem Kokosfettsäuremonoglycerid und einem Talgalkohol+40 EO-Addukt (Gewichtsverhältnis 1 : 1) eingesetzt. Es resultierte eine hellgelbgefärbte, wachsartige Masse, die sich leicht zu Schuppen verarbeiten ließ.

### Beispiele 5, 6 und 7

Beispiel 1 wurde wiederholt, anstelle der Gluconsäure jedoch jeweils 0,3 mol Ascorbinsäure, Glucarsäure oder Glucuronsäure eingesetzt. Es resultierte in allen drei Fällen wiederum dunkelgefärbte Pasten.

### Beispiele 8 und 9

Beispiel 1 wurde wiederholt, jedoch anstelle des Dimethylsulfats jeweils 0,5 mol Methylchlorid bzw. Diethylcarbonat eingesetzt. Es resultierten in beiden Fällen wiederum dunkelgefärbte Pasten.

### Beispiel 10

Beispiel 1 wurde wiederholt, 285 g (0,4 mol) des rohen Fettsäure/Gluconsäuretriethanolaminesters in einen Autoklaven überführt und mit 5 g - entsprechend 2,5 Gew.-% bezogen auf den Ester - Natriummethylat in Form einer 30 Gew.-%igen methanolischen Lösung versetzt. Der Autoklav wurde dreimal abwechselnd evakuiert und wieder mit Stickstoff beaufschlagt, auf 125°C erhitzt und dann portionsweise mit 44 g (1 mol) Ethylenoxid beaufschlagt. Nach dem Ende der Zugabe ließ man weitere 30 min nachrühren, dann wurde der Autoklav abgekühlt und entspannt. Der ethoxylierte Triethanolaminester wurde anschließend wie in Beispiel 1 beschrieben quaterniert. Es wurde eine hellgelbgefärbte, fließfähige Paste erhalten.

### II. Anwendungsbeispiele Haarkosmetik

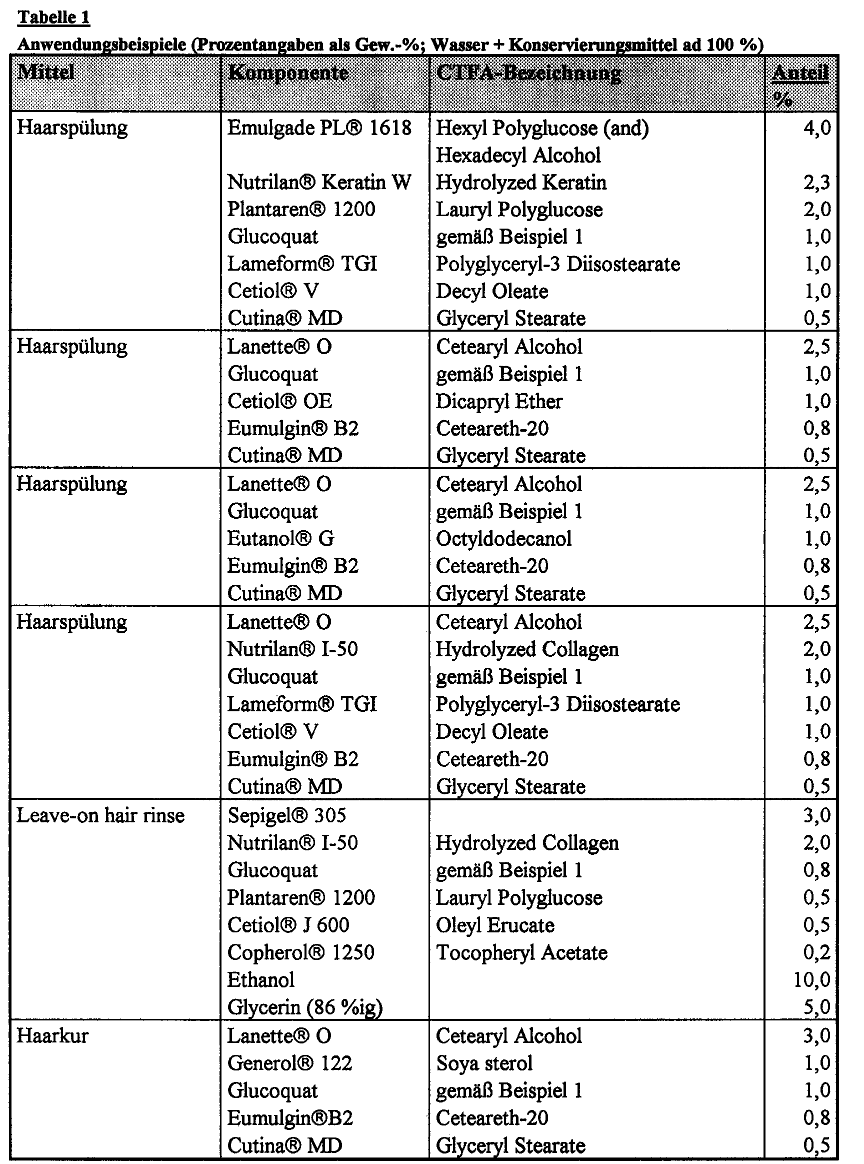

**Tabelle 1B**

| **Anwendungsbeispiele Haarkosmetik (Forts.)** | | | |
|---|---|---|---|
| **Mittel** | **Komponente** | **CTFA-Bezeichnung** | **Anteil %** |
| Haarkur | Lanette® O | Cetearyl Alcohol | 2,5 |
| | Glucoquat | gemäß Beispiel 1 | 1,5 |
| | Eumulgin® B2 | Ceteareth-20 | 1,0 |
| | Generol® 122 | Soya sterol | 1,0 |
| | Eutanol® G | Octyldodecanol | 1,0 |
| | Cutina® MD | Glyceryl Stearate | 0,5 |
| Duschbad | Texapon® K 14 S | Sodium Myreth Sulfate | 38,0 |
| | Plantaren® 2000 | Decyl Polyglucose | 7,0 |
| | Lamesoft® LMG | Glyceryl Laurate (and) Potassium Cocoyl Hydrol. Collagen | 3,0 |
| | Arylpon® F | Laureth-2 | 3,0 |
| | Glucoquat | gemäß Beispiel 1 | 0,5 |
| Duschbad | Texapon® NSO | Sodium Laureth Sulfate | 38,0 |
| | Plantaren® 2000 | Decyl Polyglucose | 7,0 |
| | Euperlan® PK 3000 | Glycol Distearate (and) Cocoamidopropyl Betaine | 3,0 |
| | Arlypon® F | Laureth-2 | 3,0 |
| | Lamesoft® LMG | Glyceryl Laurate (and) Potassium Cocoyl Hydrol. Collagen | 2,0 |
| | Glucoquat | gemäß Beispiel 1 | 0,5 |
| | Kochsalz | | 1,5 |
| Duschgel | Texapon® NSO | Sodium Laureth Sulfate | 25,0 |
| | Texapon® SB3 | Disodium Laurethsulfosuccinate | 10,0 |
| | Dehyton® K | Cocoamidopropyl Betaine | 10,0 |
| | Plantaren® 2000 | Decyl Polyglucose | 6,0 |
| | Euperlan® PK 3000 | Glycol Distearate (and) Cocoamidopropyl Betaine | 5,0 |
| | Lamesoft® LMG | Glycol Distearate (and) Cocoamidopropyl Betaine | 4,0 |
| | Cetiol® HE | PEG-7 Glyceryl Cocoate | 1,0 |
| | Arlypon® F | Laureth-2 | 1,0 |
| | Glucoquat | gemäß Beispiel 1 | 0,5 |
| Waschlotion | Plantaren® PS 10 | Sodium Laureth Sulfate (and) Lauryl Polyglucose | 16,0 |
| | Euperlan® PK 3000 | Glycol Distearate (and) Cocoamidopropyl Betaine | 5,0 |
| | Glucoquat | gemäß Beispiel 1 | 0,5 |
| | Kochsalz | | 1,5 |

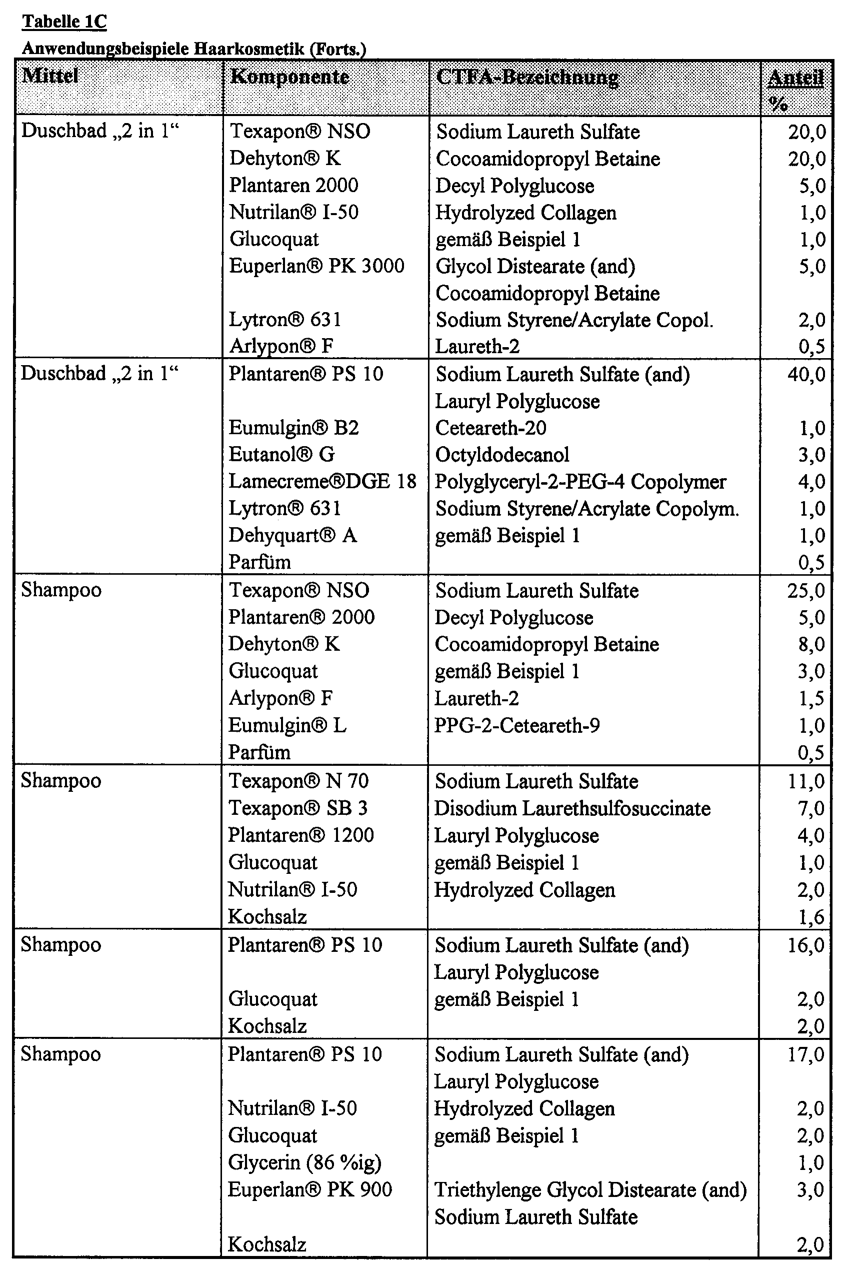

**Tabelle 1D**

| **Anwendungsbeispiele Haarkosmetik (Forts.)** | | | |
|---|---|---|---|
| **Mittel** | **Komponente** | **CTFA-Bezeichnung** | **Anteil %** |
| Shampoo | Texapon® ALS | Ammonium Laureth Sulfate | 23,0 |
| | Plantaren® 2000 | Decyl Polyglucose | 4,0 |
| | Dehyton® K | Cocoamidopropyl Betaine | 7,0 |
| | Glucoquat | gemäß Beispiel 1 | 2,0 |
| | Lamesoft® 156 | Hydrogenated Tallow Glycerides | 5,0 |
| | Monomuls® 90-L 12 | Glyceryl Laurate | 1,0 |
| | Kochsalz | | 3,0 |
| Schaumbad | Plantaren® PS 10 | Sodium Laureth Sulfate (and) Lauryl Polyglucose | 22,0 |
| | Dehyton® K | Cocoamidopropyl Betaine | 15,0 |
| | Glucoquat | gemäß Beispiel 1 | 3,0 |
| | Cetiol® HE | PEG-7 Glyceryl Cocoate | 2,0 |
| | Euperlan® PK 3000 | Glycol Distearate (and) Cocoamidopropyl Betaine | 5,0 |
| Schaumbad | Texapon® NSO | Sodium Laureth Sulfate | 30,0 |
| | Dehyton® K | Cocoamidopropyl Betaine | 10,0 |
| | Plantaren® 1200 | Lauryl Polyglucose | 10,0 |
| | Lamesoft® LMG | Glyceryl Laurate (and) Potassium Cocoyl Hydrol. Collagen | 4,0 |
| | Glucoquat | gemäß Beispiel 1 | 2,0 |
| | Guadin® AGP | Hydrolyzed Wheat Protein | 0,5 |
| Schaumbad | Melissenöl | | 5,0 |
| | Eumulgin® L | PPG-2-Ceteareth-9 | 15,0 |
| | Plantaren® 2000 | Decyl Polyglucose | 30,0 |
| | Dehyton® K | Cocoamidopropyl Betaine | 10,0 |
| | Glucoquat | gemäß Beispiel 1 | 4,0 |
| | Propylenglycol | | 4,0 |
| | Arlypon® F | Laureth-2 | 1,5 |

## Patentansprüche

1. Glucoquats, dadurch erhältlich, daß man Trialkanolamine mit einer Mischung aus Zuckersäuren und Fettsäuren umsetzt, die resultierenden Ester gegebenenfalls alkoxyliert und anschließend in an sich bekannter Weise quaterniert.

2. Verfahren zur Herstellung von Glucoquats, bei dem man Trialkanolamine mit einer Mischung aus Zuckersäuren und Fettsäuren umsetzt und die resultierenden Ester gegebenenfalls alkoxyliert und anschließend in an sich bekannter Weise quaterniert.

3. Verfahren nach Anspruch 2, **dadurch gekennzeichnet,** daß man Triethanolamin einsetzt.

4. Verfahren nach den Ansprüchen 2 und 3, **dadurch gekennzeichnet,** daß man Zuckersäuren einsetzt, die ausgewählt sind aus der Gruppe, die gebildet wird von Gluconsäure, Ascorbinsäure und Glucuronsäure.

5. Verfahren nach den Ansprüchen 2 bis 4, **dadurch gekennzeichnet,** daß man Fettsäuren der Formel (**I**) einsetzt, 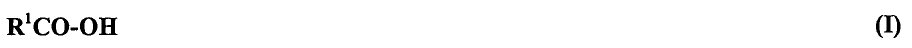 in der R¹CO für einen aliphatischen, linearen oder verzweigten Acylrest mit 6 bis 22 Kohlenstoffatomen und 0 und/oder 1, 2 oder 3 Doppelbindungen steht.

6. Verfahren nach den Ansprüchen 2 bis 5, **dadurch gekennzeichnet,** daß man die Zuckersäuren und die Fettsäuren im molaren Verhältnis 1 : 10 bis 10 : 1 einsetzt.

7. Verfahren nach den Ansprüchen 2 bis 6, **dadurch gekennzeichnet,** daß man die Trialkanolamine einerseits und die Summe aus Zucker- und Fettsäuren andererseits im molaren Verhältnis 1 : 1,3 bis 1 : 2,4 einsetzt.

8. Verfahren nach den Ansprüchen 2 bis 7, **dadurch gekennzeichnet,** daß man die Veresterung in Gegenwart von hypophosphoriger Säure bzw. deren Alkalisalzen durchführt.

9. Verfahren nach den Ansprüchen 2 bis 8, **dadurch gekennzeichnet,** daß man zunächst eine partielle Veresterung der Trialkanolamine mit den Zuckersäuren durchführt und anschließend die Fettsäuren zugibt.

10. Verfahren nach den Ansprüchen 2 bis 9, **dadurch gekennzeichnet,** daß man Alkylierungsmittel einsetzt, die ausgewählt sind aus der Gruppe, die gebildet wird von Alkylhalogeniden, Dialkylsulfaten und Dialkylcarbonaten.

11. Verfahren nach den Ansprüchen 2 bis 10, **dadurch gekennzeichnet,** daß man die Quaternierung in Gegenwart von Dispergatoren bzw. Emulgatoren durchführt, die ausgewählt sind aus der Gruppe, die gebildet wird von Fettalkoholen, Polyolen, Partialglyceriden, anionischen Tensiden und nichtionischen Tensiden.

12. Verwendung von Glucoquats nach Anspruch 1 zur Herstellung von oberflächenaktiven Mitteln.
